Europäisches Patentamt

European Patent Office

Office européen des brevets

(11) Publication number: **0 440 759 A1**

## (12) EUROPEAN PATENT APPLICATION
### published in accordance with Art. 158(3) EPC

(21) Application number: 90911713.7

(51) Int. Cl.⁵: **A61N 2/08**

(22) Date of filing: **31.07.90**

(86) International application number:
**PCT/ES90/00026**

(87) International publication number:
**WO 91/01775 (21.02.91 91/05)**

(30) Priority: **31.07.89 ES 8902700**

(43) Date of publication of application:
**14.08.91 Bulletin 91/33**

(84) Designated Contracting States:
**AT BE CH DE DK FR GB IT LI LU NL SE**

(71) Applicant: **BOBIS ZAPICO, Emilio**
**Castello, 36**
**E-28001 Madrid(ES)**

(72) Inventor: **BOBIS ZAPICO, Emilio**
**Castello, 36**
**E-28001 Madrid(ES)**

(74) Representative: **Carpintero Lopez, Francisco**
**HERRERO & ASOCIADOS, S.L. Alcalá, 21**
**E-28014 Madrid(ES)**

(54) **ASSEMBLY OR PLATE OF MAGNETS HAVING A THERAPEUTIC ACTION.**

(57) Assembly or plate of magnets (1,2,4,5,6), configured by joining a group of magnets (1,2,4,5,6), preferably five magnets, thereby forming at least seven symbols, said magnets (1,2,4,5,6) being integral, when uniting in one of its faces the north pole and on the opposite face the south pole, the same pole not being on the same surface, thereby providing an alternate polarity. There is also provided another proximate group of three magnets in longitudinal projection, also with alternate polarity.

FIG.-1

## OBJECT OF THE INVENTION

The present invention relates to a magnet assembly or plate, essentially having therapeutic action, which is comprised by a group of magnets the number whereof may vary, although the preferred number is five, which magnets are of different shapes and sizes, some adopting symmetrical geometric shapes, some having their North polarity directed upwards, and others vice versa, to form at least seven different symbols.

## BACKGROUND OF THE INVENTION

Reason for its therapeutic action. The human body is full of electricity and magnetism, as is generally known, and also of radioactivity. Specifically speaking, pain is an electromagnetic fluid and is likewise the fluid which causes pain in non-viral diseases, as shown by our experiences carried out on more than 8000 people. Many diseases are originated by the unbalancing of the radioelectromagnetic system, either as a result of harmful electric ions or other failures in the circuits. The nature of the plate magnetism attracts harmful magnetism such as pain and in other cases returns the system to a state of electrical equilibrium, even in the absence of pain.

It is well known that many diseases which affect the human race are due to unbalances of an electromagnetic type, wherein the most largely used healing process is acupuncture, digitopuncture or, to a lesser extent, the placing of magnets near the area where the disease or point of healing thereof is located, thus restoring magnetic balance to the body.

## DESCRIPTION OF THE INVENTION

The magnet assembly or plate object of the invention is comprised by a group of magnets, the number whereof may vary, although for reasons of effectiveness the preferred number is five, which magnets represent at least seven of the symbols which are generally accepted by orthodox teachings.

Amongst such symbols the following can be pointed out:

(▲) TRIANGLE: The triangle represents Fertilization, Divinity and the Trinity.

(Y): Represents generation, sexual union or copulation, the free decision of each person to follow the straight or the crooked path, dualism, ˜etc.

(α) ALPHA: Represents the beginning of all things.

(Ω) OMEGA: Represents the end of all things towards infinity. Together with the previous symbol, it represents the beginning and end, God and Son, in other words, everything.

(T): Represents the force of creation or masculinity, Tau, Theos, God. Some civilizations even used this symbol to represent the cross.

(H): Symbol of inspiration, Homo.

(I): Number one of the symbol of wealth, unity, of life and the beginning of activity, solidity, supreme hierarchy, the mast, the vessel.

It should be pointed out that the effectiveness obtained by this plate is not directly related to a greater strength of the magnets which constitute same, but rather such strength must be relative and proportional, and its effectiveness lies in the correct arrangement of the magnets, with the suitable polarity facing the respective surface.

For application to Medicine and Physics, the said assembly or plate of magnets should, for greater effectiveness, carry the symbol of the latin Cross, also with alternate polarity, arranged as in the previous assembly, but with no other magnet. It has likewise been observed that the plate is more effective when the magnet assemblies are placed two by two, one adjacent the other, but inverted in their arrangement and therefore as regards their polarity.

This plate or assembly of magnets has excellent properties, since it can provide almost instant relief from pain, and alleviate certain diseases, including those of the sight.

## DESCRIPTION OF THE DRAWINGS

In order to complete the description of the magnet plate or assembly being made, and to achieve a better understanding of the characteristics of the invention, a set of drawings is attached to the present specification, as an integral part thereof, wherein the following has been shown in an illustrative and non-limiting manner:

Figure 1 shows the magnet assembly or plate which constitutes the object of the invention.

Figure 2 shows the preferred arrangement of the magnet plates for application to Medicine.

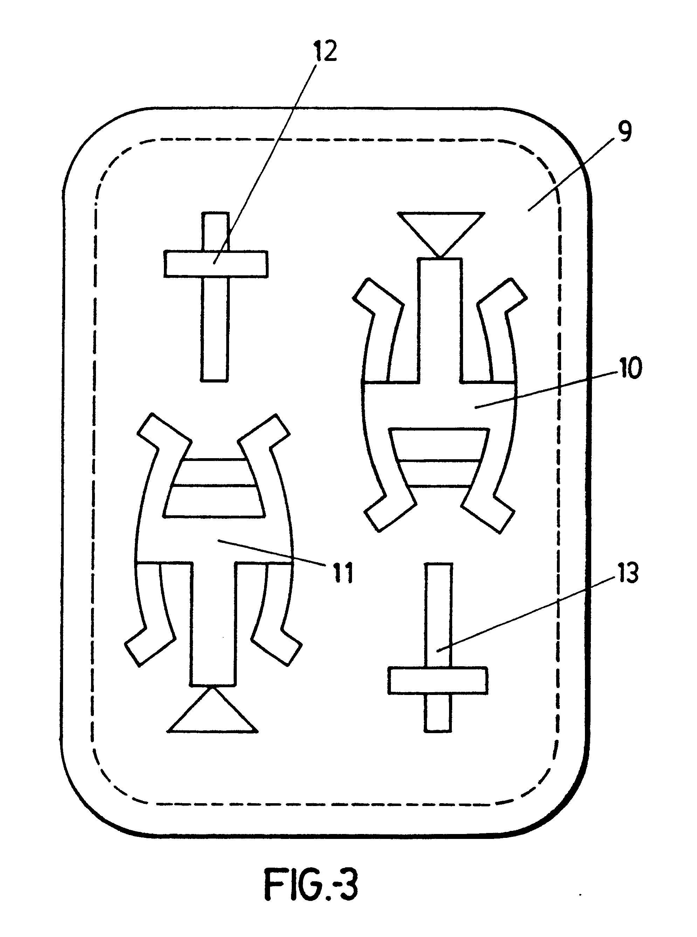

Figure 3 shows a practical embodiment for the user, wherein the assembly of figure 2 is repeated in a different position.

## PREFERRED EMBODIMENT OF THE INVENTION

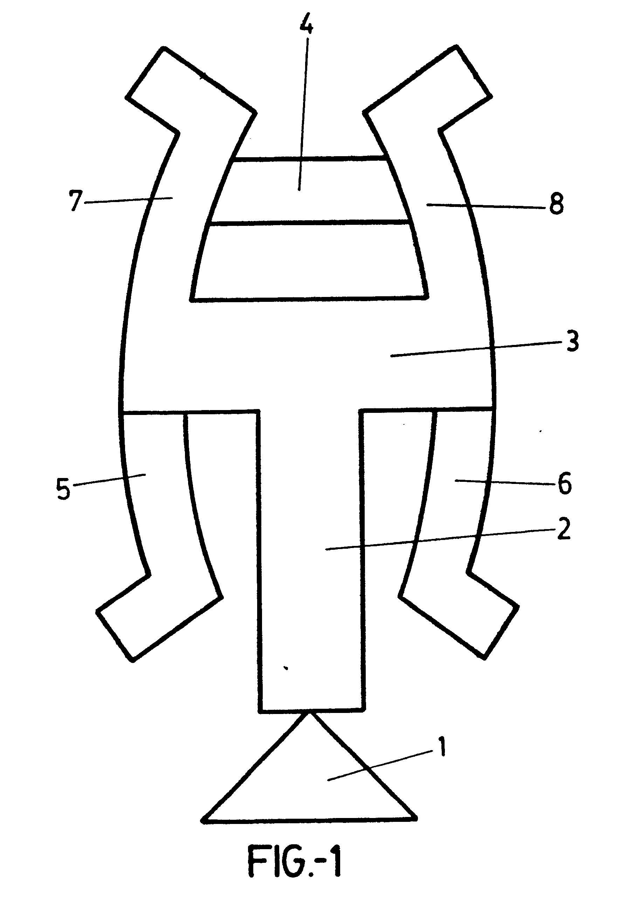

Figure 1 shows the magnet assembly or plate comprising the invention, where the seven symbols contained therein are referenced as follows:

(▲) TRIANGLE:

Represented by lower portion (1).

(Y):

Obtained by means of arms (2), (3), (4) and (5).

(α) ALPHA:

Obtained by means of arms (3), (4), (7) and (8).

(Ω) OMEGA:

Obtained by means of arms (5), (3) and (6).

(T):

Obtained by means of arms (2) and (3).

(H):

Obtained by means of arms (5), (6), (7) and (8).

(I):

Obtained by means of arm (2).

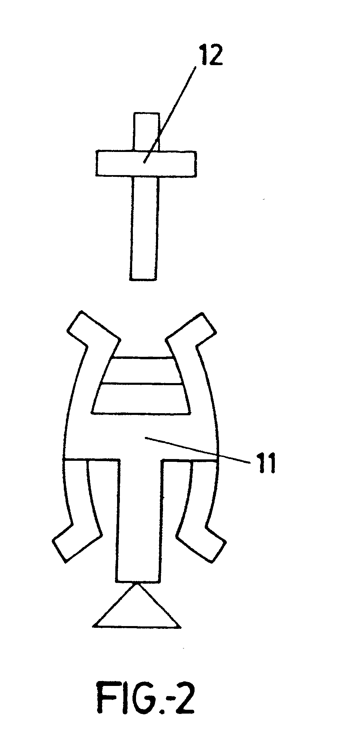

Figure 2 shows a preferred arrangement of the magnet plate for application to Medicine. The shapes of plate assemblies (10) and (11) are stamped on a base plate (9), as are the elements in the shape of a cross (12) and (13), arranged, as can be seen, in opposite orientations. The bodies of the magnets are placed in the depressions thus formed, whereby possible movement or displacement thereof is prevented.

The magnets are in a single piece, the North pole being on one surface and the South pole on the other. Not all of the magnets need have the same pole on the same surface, but rather polarity alternates, in other words, whilst some show their North pole, the adjacent magnets show their South pole.

This plate has a further property in Physics: it attracts a magnetic wheel, passes thereover and projects it in the same direction, this being a necessary and essential property for its therapeutic action.

## Claims

1. Magnet assembly or plate having therapeutic action, characterised in that it is comprised by a group of magnets, preferrably five in number, to form at least seven symbols, the said magnets being of a single piece, having the north pole on one of their surfaces and the south pole on the other, the whole group of magnets not having the same polarity on the same surface, but rather such polarity alternates, one magnet showing its north pole and the adjacent magnet its south pole, the application of which plate on any point of the human body alleviates and gives relief from pain.

2. Magnet assembly or plate having therapeutic action, in accordance with claim 1, characterised in that the symbols comprised within the plate are the Triangle, representing Fertilization, Divinity and the Trinity, the symbol of the Greek letter Y, representing generation, sexual union or freedom of choice, ALPHA representing the beginning and OMEGA representing the end, the symbol T representing the force of creation, the symbol H representing inspiration, and the symbol I as number 1, representing union and wealth.

3. Magnet assembly or plate having therapeutic action, in accordance with claims 1 and 2, characterised in that the action and effectiveness thereof is very closely related to the type of magnetism produced by the different symbols and the position of their polarity, rather than to a greater magnetic strength thereof.

4. Magnet assembly or plate having therapeutic action, in accordance with claims 1 to 3, characterised in that for use in Medicine these two magnet assemblies or plates will be arranged on a common base plate which comprise same and prevents their displacement, together with two further elements in the shape of a cross which are placed in opposite orientations, thereby considerably improving their curative action.

5. Magnet assembly or plate having therapeutic action, in accordance with the preceding claims, characterised in that in addition to its therapeutic properties, the plate has physical properties which had never before been achieved, namely the attraction of a magnetic wheel, passage thereover and projection of the said wheel in the same direction.

6. Magnet assembly or plate having therapeutic action, in accordance with the preceding claims, characterised in that for optimum effectiveness, both in Physics and in Medicine, same should be joined to a further assembly of three magnets which make up the symbol of the Cross, as shown in the drawings, likewise with alternate polarities.

FIG.-1

FIG.-2

FIG.3

# INTERNATIONAL SEARCH REPORT

International Application No PCT/ES 90/00026

**I. CLASSIFICATION OF SUBJECT MATTER** (if several classification symbols apply, indicate all) *

According to International Patent Classification (IPC) or to both National Classification and IPC

Int.Cl.$^5$   A 61 N 2/08

**II. FIELDS SEARCHED**

| Minimum Documentation Searched [7] | |
|---|---|
| Classification System | Classification Symbols |
| Int.Cl.$^5$ | A 61 N |

Documentation Searched other than Minimum Documentation
to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | FR, A, 2580180 (RAZE) 17 October 1986, see the whole document | 1,3 |
| A | | 2 |
| A | FR, A, 2579105 (CASELLI) 26 September 1986, see the whole document | 1,6 |
| A, P | EP, A, 0334141 (HOLCOMB) 27 September 1989, see page 3, line 16 - page 4, line 4; figure 3 | 1,4 |
| A | FR, A, 2054766 (WAGRET) 7 May 1971, see the whole document | 1,2 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 25 October 1990 (25.10.90) | 15 November 1990 (15.11.90) |
| International Searching Authority | Signature of Authorized Officer |
| | |

Form PCT/ISA/210 (second sheet) (January 1985)

**FURTHER INFORMATION CONTINUED FROM THE SECOND SHEET**

**V.☐ OBSERVATIONS WHERE CERTAIN CLAIMS WERE FOUND UNSEARCHABLE** [1]

This international search report has not been established in respect of certain claims under Article 17(2) (a) for the following reasons:

1.☒ Claim numbers ....5...., because they relate to subject matter not required to be searched by this Authority, namely:

Rule.39. 1 (i) : scientific and mathematical theories
and
Rule.39. 1 (V) : mere presentations of information

2.☐ Claim numbers ..........., because they relate to parts of the international application that do not comply with the prescribed requirements to such an extent that no meaningful international search can be carried out. specifically:

3.☐ Claim numbers .........., because they are dependent claims and are not drafted in accordance with the second and third sentences of PCT Rule 6.4(a).

**VI.☐ OBSERVATIONS WHERE UNITY OF INVENTION IS LACKING** [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

**Remark on Protest**

☐ The additional search fees were accompanied by applicant's protest.

☐ No protest accompanied the payment of additional search fees.